(19) Europäisches Patentamt
European Patent Office
Office européen des brevets

(11) Veröffentlichungsnummer: **0 010 142**
**B1**

(12) # EUROPÄISCHE PATENTSCHRIFT

(45) Veröffentlichungstag der Patentschrift: **22.07.81**

(51) Int. Cl.³: **C 07 D 307/06,** C 07 D 307/12, A 23 L 1/235, A 61 K 7/46, C 11 B 9/00

(21) Anmeldenummer: **79103123.0**

(22) Anmeldetag: **24.08.79**

(54) **Als Einzelverbindungen oder in Form von Gemischen vorliegende sesquiterpenoide Tetrahydrofuranderivate (I), Verfahren und Ausgangsstoffe (V) zu deren Herstellung, Verwendung von (I) als Riech- und/oder Geschmackstoffe und Riech- und/oder Geschmackstoffkompositionen mit einem Gehalt an (I).**

(30) Priorität: **08.09.78 CH 9470/78**

(43) Veröffentlichungstag der Anmeldung: **30.04.80 Patentblatt 80/9**

(45) Bekanntmachung des Hinweises auf die Patenterteilung: **22.07.81 Patentblatt 81/29**

(84) Benannte Vertragsstaaten: **CH DE FR GB NL**

(56) Entgegenhaltungen:
**DE-B-2 143 232**
**GB-A-1 311 408**

(73) Patentinhaber: **L. Givaudan & Cie Société Anonyme, Patentdienst Postfach, CH-4002 Basel (CH)**

(72) Erfinder: **Kaiser, Roman, 90 East Fourth Street, Clifton, N.J. 07011 (US)**
Erfinder: **Lamparsky, Dietmar, Dr., Sonnhalde 8, CH-8602 Wangen-Dübendorf (CH)**

(74) Vertreter: **Urech, Peter, Dr., et al, Grenzacherstrasse 124, Postfach 601, CH-4002 Basel (CH)**

EP 0 010 142 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Als Einzelverbindungen oder in Form von Gemischen vorliegende sesquiterpenoide Tetrahydrofuranderivate (I), Verfahren und Ausgangsstoffe (V) zu deren Herstellung, Verwendung von (I) als Riech- und/oder Geschmackstoffe und Riech- und/oder Geschmackstoffkompositionen mit einem Gehalt an (I)

Die Erfindung betrifft neue Riech- und/oder Geschmackstoffe. Es handelt sich dabei um die Verbindungen der Formel

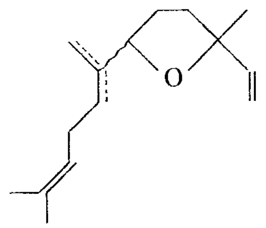

(I)

worin eine der gestrichelt gezeichneten Linien eine zusätzliche Bindung darstellt.

Die Formel soll demgemäß einerseits alle 4 Isomeren des 2-Methyl-2-vinyl-5-(1'-methylen-5'-methylhex-4'-en-1'-yl)-tetrahydrofurans, d. h. alle 4 Enantiomeren der beiden diastereoisomeren Formen Ia und Ib umfassen.

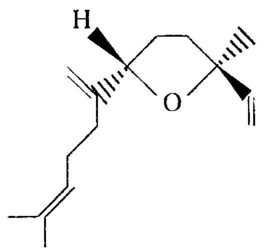

(Ia)

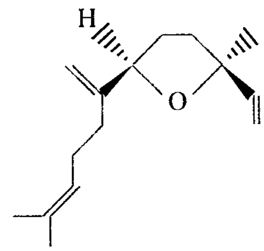

(Ib)

Andererseits soll die Formel I sämtliche 8 Stereoisomeren des 2-Methyl-2-vinyl-5-(1',5'-dimethylhexa-1',4'-dien-1'-yl)-tetrahydrofurans, d. h. die 8 Enantiomeren der 4 diastereoisomeren Formen Ic bis If umfassen:

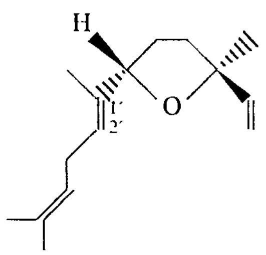

(Ic)

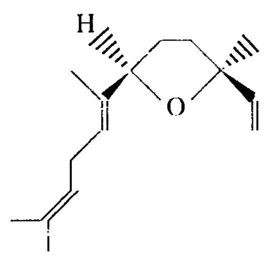

(Id)

| | (Ic) | (Id) |
|---|---|---|
| Seitenkette/ Vinylgruppe | trans | cis |
| $C_{1'}/C_{2'}$ | trans | trans |

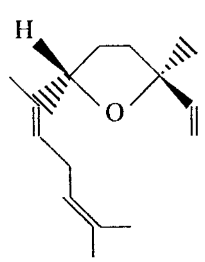

(Ie)

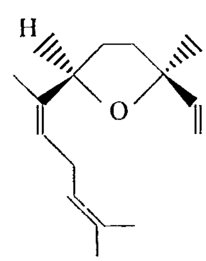

(If)

| | (Ie) | (If) |
|---|---|---|
| Seitenkette/ Vinylgruppe | trans | cis |
| $C_{1'}/C_{2'}$ | cis | cis |

Die Erfindung betrifft ferner ein Verfahren zur Herstellung der Verbindungen der Formel I.

Dieses Verfahren ist dadurch gekennzeichnet, daß man 2-Methyl-2-vinyl-5-(1'-hydroxy[bzw. acyloxy]-1',5'-dimethyl-hex-4'-en-1'-yl)-tetrahydrofuran dehydratisiert bzw. deacyliert.

Bei diesem erfindungsgemäßen Verfahren wird also die Hydroxyl- bzw. Acetoxy-Sauerstoffunktion in Form einer $\beta$-Eliminierungsreaktion entfernt.

Als Ausgangsmaterial kann beispielsweise das Isomerengemisch II bzw. können die einzelnen Isomeren IIa—IId von 2-Methyl-2-vinyl-5-(1'-hydroxy-1',5'-dimethyl-hex-4'-en-1'-yl)-tetrahydrofuran

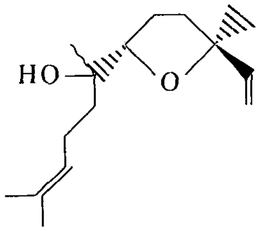

(IIa)/(IIb)

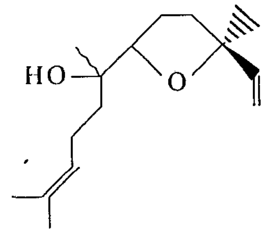

(IIc/IId)

eingesetzt werden.

Diese einzelnen Isomeren von II bzw. das Isomerengemisch können (kann) mit Hilfe von geeigneten Reagenzien direkt zu I dehydratisiert werden.

Als Dehydratisierungsagenzien eignen sich beispielsweise:

Phosphoroxyhalogenide, wie Phosphoroxychlorid, in Anwesenheit eines Überschusses an einer Base wie Pyridin, Thionylchlorid, in Anwesenheit eines Überschusses an einer Base wie Pyridin, organische und anorganische Säuren wie p-Toluolsulfonsäure, Oxalsäure, Salzsäure usw., saure Salze wie Kaliumbisulfat usw.

Auf diese Weise fällt I als Isomerengemisch von Ia—If an, bzw. im Falle des Diastereomerenpaares IIa/IIb als Ausgangsmaterial als Gemisch Ia, Ic und Ie und im Falle von IIc/IId als Ausgangsmaterial als Gemisch Ib, Id und If. Ie bzw. If sind in den Produktgemischen jeweils in nur geringer Menge enthalten, ihre Anwesenheit ergibt sich aber aus der säulen- und gaschromatographischen Aufarbeitung des Gemisches eindeutig anhand der gaschromatographischen Analyse (Schulter auf Ia bzw. Ib auf gepackten Kolonnen oder als getrennte peaks in Glaskapillarsäulen) und dem damit verbundenen Auftreten charakteristischer Signale im NMR-Spektrum der isolierten Proben von Ia bzw. Ib.

Vorzugsweise wird II aber zunächst in einen Säureester übergeführt, also beispielsweise in das Acetat, Propionat, Butyrat oder Benzoat, und hierauf die den Alkoholen II entsprechenden Ester IIIa—IIId bzw. deren Gemische deacyliert, zweckmäßigerweise dadurch, daß sie der Pyrolyse unterworfen werden.

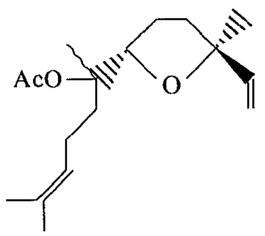

(IIIa)/(IIIb)

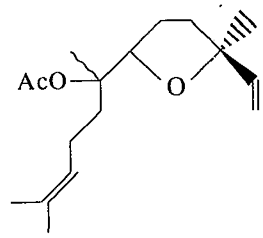

(IIIc)/(IIId)

Ac = nieder-Alkanoyl, Aroyl

Die Veresterung kann nach an sich bekannten Methoden, z. B. durch Behandeln von II mit einem reaktionsfähigen Säurederivat von AcOH, wie einem Halogenid oder dem Anhydrid durchgeführt werden. Man arbeitet dabei in Anwesenheit einer Base, wie Dimethylanilin, Diäthylanilin, Pyridin etc. III wird hierauf zu I pyrolysiert.

Die Pyrolyse von III kann z. B. bereits durch einfaches Erhitzen der einzelnen Ester oder des Estergemisches in einem Hickmann-Kolben in Stickstoffatmosphäre, beispielsweise auf 260—300°C bewerkstelligt werden. Das dabei entstehende saure Destillat kann in einem Lösungsmittel, z. B. einem Äther aufgenommen, mit Bicarbonatlösung und Wasser bis zum Neutralpunkt gewaschen, getrocknet und eingeengt werden.

Bessere Resultate werden erhalten, wenn das zu pyrolysierende Material als ca. 20—40, z. B. 30%ige Lösung in Hexan, Benzol etc. in ein auf eine Temperatur zwischen ca. 300—500°C, insbesondere ca. 370—430°C konditioniertes Pyrolyserohr — welches zweckmäßiger Weise mit Glasraschigringen beschickt ist — getropft wird. Aber auch andere Trägermaterialien, wie Keramikfüllkörper, Eisen- oder

Kupfernetzchen usw. kommen in Frage.

Die Verbindungen II und III sind neu und bilden ebenfalls Gegenstand der vorliegenden Erfindung.

Als Ausgangsmaterialien zur Herstellung von II können die trans- und cis- aus der Literatur bekannten (vgl. A. F. Thomas, R. Dubini, Helv. 57, 2066 [1974]) 2-Methyl-2-vinyl-5-acetyl-tetrahydrofurane IVa und IVb verwendet werden.

Die Grignard-Reaktion von beispielsweise 4-Methyl-3-penten-1-yl-magnesiumbromid (F. Medina, A. Manjarrez, Tetrah. 20, 1807 [1964]) mit IVa bzw. IVb oder mit dem bei der Synthese von IV normalerweise anfallenden 2 : 1-Gemisch von IVa/IVb führt zu den Diastereoisomerengemischen der 2-Methyl-2-vinyl-5-(1'-hydroxyl-1',5'-dimethyl-hex-4'-en-1'-yl)-tetrahydrofurane IIa/IIb bzw. IIc/IId im Verhältnis von jeweils 9 : 1, bzw. zum Gemisch von IIa, IIb, IIc, IId im Verhältnis von 18 : 1 : 9 : 1.

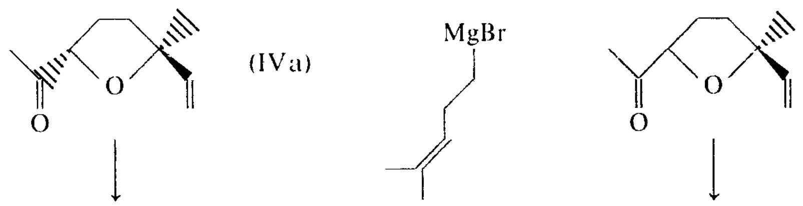

(IVa) (IVb)

(IIa)/(IIb) (9:1) (IIc)/(IId) (9:1)

Die Trennung der Isomerengemische [von I, II und III oder IV], kann auf übliche Weise, z. B. mittels Säulenchromatographie oder präparativer Gaschromatographie erfolgen. Wie weiter unten ersichtlich, unterscheiden sich die Isomeren von I in ihren organoleptischen Eigenschaften nicht grundlegend, so daß aus wirtschaftlichen Gründen insbesondere das Isomerengemisch verwendet werden kann.

Die Verbindungen I weisen besondere organoleptische Eigenschaften auf, auf Grund derer sie sich vorzüglich als Riech- und/oder Geschmackstoffe eignen.

Die Erfindung betrifft demgemäß auch die Verwendung der Verbindungen I, insbesondere in praktisch reiner Form oder in Form von Gemischen, mit Ausnahme von natürlichen, I enthaltenden Gemischen, als Riech- und/oder Geschmackstoffe.

Unter praktisch reinen I sollen insbesondere solche I verstanden werden, die frei von Begleitstoffen sind, wie sie neben I in natürlichen Extrakten vorliegen. Als praktisch reine I im Sinne der vorliegenden Erfindung sollen insbesondere synthetisch hergestellte I verstanden werden.

Die natürlichen, I enthaltenden Gemische sollen darum ausgeschlossen sein, da im Zuge der vorliegenden Arbeiten gefunden wurde, daß Ic/Id, und zwar im Verhältnis von ca. 1 : 1 zu $<0{,}01\%$ im absoluten Öl von Osmanthus fragrans Lour. enthalten sind.

Die erfindungsgemäß als Riech- und/oder Geschmackstoffe verwendeten Verbindungen I zeichnen sich durch einen frischen, fruchtigen, sehr natürlich wirkenden Geruch aus, der auch an gewisse Aspekte von Äpfeln, Birnen und jungen Pflanzenknospen erinnert. Die einzelnen Isomeren riechen annähernd gleich, außer daß bei den Isomeren Ia und Ib eine zusätzliche terpenig wirkende Note feststellbar ist. Wirtschaftlichen Überlegungen zufolge wird daher mit Vorteil das Isomerengemisch verwendet. Die Verbindungen können demgemäß beispielsweise zur Parfümierung bzw. Aromatisierung von Produkten, wie Kosmetika (Seifen, Salben, Pudern, Zahnpasten, Mundwässern, Desodorantien, Shampoos, Lotionen, etc.), Detergentien, bzw. Nahrungsmitteln, Genußmitteln und Getränken dienen, wobei die Verbindungen vorzugsweise nicht allein, sondern in Form von Kompositionen mit andern Riech- bzw. Geschmackstoffen eingesetzt werden. Solche Riech- bzw. Geschmackstoffkompositionen mit einem Gehalt an Verbindungen I bilden ebenfalls Gegenstand der Erfindung.

Ihre Herstellung erfolgt auf an sich bekannte Art und Weise (Zugabe von I zu bekannten Riech- bzw. Geschmackstoffkompositionen oder Vermischung von I mit als Bestandteil von Riech- bzw. Geschmackstoffkompositionen geeigneten natürlichen oder synthetischen Verbindungen oder Gemischen).

Als Riechstoffe eignen sich die Verbindungen I auf Grund ihrer oben beschriebenen, originellen Noten, insbesondere in Kombination mit einer Reihe von natürlichen und synthetischen Riechstoffen wie z. B.

- Galbanumöl, Mastixöl, Vetiveröl, Patchouliöl, Sandelholzöl, Mandarinenöl, Petitgrainöl, Ylang-Ylang-Öl, Basilikumöl, Baummoos absolue, Patchouliblätteröl, Cedernöl, Fichtenöl, Lorbeeröl, Costuswurzelöl, Calmusöl, Beifußöl, Kamillenöl, Wermutöl, Wurmsamenöl, Selleriesamenöl, Angelikasamenöl, Sternanisöl, Thymianöl, Rosmarinöl, Lavendelöl, Lavandinöl, Aspiköl, Salbeiöl, Neroliöl, Bergamottöl, Citronenöl, Orangenöl, Grapefruitöl, Geraniumöl, Benzoeresinoid, Melilotus absolue, Jasmin absolue, Rosenöl, Canangaöl, Corianderöl, Cassie absolue, Narzissen absolue, Verveine absolue oder Öl, Veilchenblätter absolue, Tuberosen absolue usw.
- Aldehyden wie Hydroxycitronellal, Zyklamenaldehyd, Benzaldehyd, p-tert.-Butyl-α-methylhydro-

zimtaldehyd, alpha-Hexylzimtaldehyd, 3,5-Dimethyl-cyclohex-3-en-1-yl-carboxaldehyd, Citral, Citronellal, 2,6-Dimethyl-5-hepten-1-al, Isovaleraldehyd, trans-2-Hexenal, Sorbinaldehyd, trans-2-Octenal, n-Octanal, n-Nonanal, trans-2-cis-6-Nonadienal, 2,4-Decadienal, Methylnonyl-acetaldehyd usw.

- Ketonen wie alpha-Ionon, betra-Inonon, Allylionon, Acetanisol, 4-(para-Hydroxyphenyl)-2-butanon, Campher, Menthon, Carvon, Pulegon usw.
- Acetalen und Ketalen wie Phenylacetaldehyd-dimethylacetal, Phenylacetaldehyd-glycerinacetal, 2-Methyl-1,3-dioxolan-2-äthylacetat, Capronaldehyd-dimethylacetal usw.
- Äthern wie Eugenolmethyläther, Methyl-1-methyl-cyclododecyläther, Anethol, Estragol usw.
- Phenolkörpern wie Vanillin, Eugenol, Isoeugenol, Creosol usw.
- Alkoholen wie Butanol, n-Hexanol, cis-3-Hexenol, trans-2, cis-6-Nonadienol, cis-6-Nonenol, Linalool, Geraniol, Nerol, Citronellol, Nerolidol, Farnesol, Benzylalkohol, Phenyläthylalkohol, Zimtalkohol usw.
- Estern wie Methyldihydrojasmonat, Linalylacetat, Bornylacetat, Geranylpropionat, Cedrylacetat, Vitiverylacetat, Äthylacetat, Isoamylacetat, Äthylbutyrat, para-tert.-Butylcyclohexylacetat, ortho-tert.-Butylcyclohexylacetat, Myraldylacetat ® (Givaudan), Benzylacetat, Benzylsalicylat, Styrallylacetat, Äthyl-$\alpha$-methylphenylglycidat, Piperonylacetat, Maltylisobutyrat, Dimethylbenzylcarbinylbutyrat, cis-3-Hexenylacetat, cis-3-Hexenylsalicylat, Äthyl-2,4-decadienoat, Äthylacetoacetat, usw.
- Lactone wie $\gamma$-Undecalacton, $\gamma$-Decalacton, $\gamma$-Nonalacton, $\delta$-Decalacton, $\delta$-Octalacton, Cumarin usw.
- Säuren wie Milchsäure, Buttersäure, $\alpha$-Methylbuttersäure, trans-2-Hexensäure, trans-2-Octansäure usw.
- Moschus- und ambraartig riechenden Körpern wie Äthylenbrassylat, 4-Acetyl-6-tert.-butyl-1,1-dimethylindan, 12-Oxahexadecanolid, 8$\alpha$,12-Oxido-13,14 15,16-tetranorlabdan usw.
- schwefelhaltigen Verbindungen wie p-Menthan-8-thiol-3-on, Dimethylsulfid und anderen Sulfiden und Disulfiden usw.
- stickstoffhaltigen Verbindungen wie Methylanthranilat, Indol, Isobutylchinolin, verschiedenen Pyrazinen, 5-Methyl-heptan-3-on-oxim usw.

Wie aus den weiter hinten folgenden Anwendungsbeispielen ersichtlich ist, lassen sich mit den neuen Tetrahydrofuranderivaten der Formel I interessante Effekte erzielen. Besonders hervorzuheben ist, daß Linalool/Linalylacetat-Gemische durch I in vorteilhafter Weise modifiziert werden, indem die entstehenden Komplexe sehr natürlich wirken und sich durch eine unerwartete Strahlungskraft auszeichnen. Da synthetisches Linalool und Linalylacetat wichtige, viel gebrauchte Parfümerieprodukte darstellen, und diese beiden Verbindungen zudem oft Hauptkomponenten in essentiellen Ölen darstellen, kommt dieser Tatsache eine beträchtliche praktische Bedeutung zu. I kann daher mit Erfolg zur Herstellung von Linalool/Linalylacetat — reichen Parfümeriekompositionen oder zur Rekonstitution von Linalool/Linalylacetat — reichen essentiellen Ölen wie z. B. der Öle der Lavendel-, Petitgrain-, Neroli-, Sauge-, Bergamottegruppe, usw. verwendet werden.

Da das Isomerengemisch der Formel I ferner Komplexe zwischen Linalool und Benzylacetat (typisch für Jasmin und andere blumige Noten), Linalool und Methylbenzoat (typisch für Richtung Ylang-Ylang), Benzylacetat und Phenyläthylalkohol (typisch für Hyazinthen-Kompositionen) und Citronellol und Phenyläthylalkohol (typisch für die Richtung Rose) usw. auf ähnliche Weise bereichert, ist es zur Modifizierung von blumigen Kompositionen oder zur Mitverwendung bei der Rekonstitution von Blütenessenzen oder -absolues vom Typ Jasmin, Rose, Hyazinthe, Narzisse, Ylang-Ylang usw. gut geeignet.

Bei der Herstellung solcher Kompositionen können die oben aufgeführten bekannten Riechstoffe nach (dem Parfumeur bekannter) Art und Weise verwendet werden, wie z. B. aus W. A. Poucher, Perfumes, Cosmetics and Soaps, Vol. 2, 7. Auflage, Chapman und Hall, London, 1974 hervorgehend.

Die Konzentration der Verbindungen I kann je nach dem Verwendungszweck innerhalb weiter Grenzen variieren, beispielsweise zwischen etwa 0,01 (Detergentien) und etwa 30 Gew.-% (alkoholische Lösungen). In Parfumbasen bzw. Konzentraten können die Konzentrationen selbstverständlich auch höher liegen. Die Parfumbasen können in üblicher Weise zur Parfümierung von Eaux de Cologne, Eaux de Toilettes, Lotionen, Crèmes, Shampoos, Seifen und Detergentien, etc. verwendet werden.

Als Geschmackstoffe können die Verbindungen I beispielsweise zur Erzeugung bzw. Verbesserung, Verstärkung, Steigerung oder Modifizierung von Frucht- oder Beerenaromen in Nahrungsmitteln (Joghurt, Süßwaren, etc.); in Genußmitteln (Tee, etc.) und Getränken (Limonaden etc.) verwendet werden.

Die ausgeprägten geschmacklichen Qualitäten von insbesondere praktisch reinen, insbesondere von synthetisch hergestellten Verbindungen I ermöglichen die Verwendung in geringen Konzentrationen. Eine geeignete Dosierung umfaßt beispielsweise den Bereich von 0,05 ppm—100 ppm, vorzugsweise von 0,1 ppm—20 ppm im Fertigprodukt, d. h. dem aromatisierten Nahrungsmittel, Genußmittel oder Getränk.

In der folgenden Tabelle sind einige Effekte zusammengestellt, wie sie sich mit den Äthern I erzielen lassen.

Tabelle

| Aroma | Dosierung | Effekt |
|---|---|---|
| Apfel | ppm im Fertigprodukt<br>0,05—100 ppm<br>insb. 0,1—10 ppm | veredelnd |
| Birne | ppm im Fertigprodukt<br>0,05—100 ppm<br>insb. 0,1—10 ppm | veredelnd |
| Aprikose,<br>Pfirsich,<br>Heidelbeere,<br>Stachelbeere | ppm im Fertigprodukt<br>0,05—100 ppm<br>insb. 0,1—10 ppm | bereichert das Aroma<br>durch Erzeugen eines<br>an die Fruchthaut<br>erinnernden Aspektes |

Die Verbindungen können auf übliche Weise mit den für Geschmackstoffkompositionen verwendeten Bestandteilen vermischt bzw. solchen Aromen zugesetzt werden. Unter den erfindungsgemäß verwendeten Aromen werden Geschmackstoffkompositionen verstanden, die sich auf an sich bekannte Art verdünnen bzw. in eßbaren Materialien verteilen lassen. Sie können nach an sich bekannten Methoden in die üblichen Gebrauchsformen, wie Lösungen, Pasten oder Pulver übergeführt werden. Die Produkte können sprühgetrocknet, vakuumgetrocknet oder lyophilisiert werden.

Die bei der Herstellung solcher Aromen zweckmäßigerweise verwendeten bekannten Aromastoffe sind entweder in der obigen Zusammenstellung bereits enthalten oder können leicht der Literatur entnommen werden, wie z. B. J. Merory, Food Flavorings, Composition, Manufacture and Use, Second Edition, The Avi Publishing Company, Inc., Westport, Conn. 1968, oder G. Fenaroli, Fenaroli's Handbook of Flavor Ingredients, Second Edition, Volume 2, CRC-Press, Inc. Cleveland, Ohio, 1975.

Für die Herstellung solcher üblicher Gebrauchsformen kommen beispielsweise folgende Trägermaterialien, Verdickungsmittel, Geschmackstoffverbesserer, Gewürze und Hilfsingredientien, etc. in Frage:

Gummi arabicum, Tragant, Salze oder Brauereihefe, Alginate, Carrageen oder ähnliche Absorbentien; Indole, Maltol, Gewürzoleoresine, Raucharomen; Gewürznelken, Natriumcitrat; Mononatriumglutamat, Dinatrium-inosin-5'-monophosphat (IMP), Dinatriumguanosin-5-phosphat (GMP); oder spezielle Aromastoffe, Wasser, Äthanol, Propylenglykol, Glycerin.

## Beispiel 1

### 2-Methyl-2-vinyl-5-(1'-hydroxy-1',5'-dimethyl-hex-4'-en-1'-yl)-tetrahydrofurane IIa/IIb und IIc/IId

Zu der Grignard-Lösung, welche aus 5,08 g (0,031 Mol) 4-Methyl-3-penten-1-yl-bromid in 20 ml Äther und 0,76 g (0,031 Mol) Magnesium-Spänen in 10 ml Diäthyläther hergestellt wird, läßt man bei 0° im Verlaufe von 20 Minuten eine Lösung von 4,0 g (0,026 Mol) trans-2-Methyl-2-vinyl-5-acetyltetrahydrofuran IVa in 10 ml Äther zutropfen. Anschließend wird während einer Stunde bei Rückflußtemperatur gerührt, dann abgekühlt und in üblicher Art aufgearbeitet. Es resultierten 4,2 g Rohprodukt, welches das Diastereoisomerenpaar IIa/IIb im Verhältnis von 9 : 1 enthält.

Analog werden aus 3,4 g (0,022 Mol) IVb 4,1 g Rohprodukt erhalten, welches das Diastereoisomerenpaar IIc/IId im Verhältnis von 9 : 1 enthält. Zur Charakterisierung werden IIa—IId mit Hilfe der präparativen Gaschromatographie in reiner Form isoliert.

Analog werden aus 98,0 g (0,64 Mol) des 2 : 1-Gemisches der 2-Methyl-2-vinyl-5-acetyltetrahydrofurane und 130,4 g (0,80 Mol) 4-Methyl-3-penten-1-ylbromid, 120 g Rohprodukt erhalten, aus welchem sich durch Destillation über eine 20-cm-Widmerkolonne 100,2 g (=66%) des Alkoholgemisches IIa—IId im Verhältnis von 18 : 2 : 9 : 1 gewinnen läßt. Sdp. 93°/0,05 Torr.

Spektrale Daten

IIa IR: 3560, 3460 (interne H-Brücke), 1640, 1180, 1150, 1120, 1090, 1050, 1020, 990, 918 $cm^{-1}$
NMR: 1,10 (3 H, s); 1,32 (3 H, s); 1,64 + 1,70 (je 3 H, s); 3,84 (1 H, m); 4,9—5,3 (2 H, AB-Teil); 5,75—6,05 (1 H, X-Teil) ~ 5,10 (1 H, dxd) δ ppm
MS: 238 ($M^+$, 1), 155 (7), 138 (28), 127 (12), 111 (27), 109 (100), 93 (30), 81 (16), 69 (88), 55 (28), 43 (74), 41 (45)

IIb IR: 3560, 3460, 1640, 1180, 1125, 1095, 1050, 1025, 990, 918 $cm^{-1}$
NMR: 1,22 (3 H, s); 1,32 (3 H, s); 1,64 + 1,70 (je 3 H, s); 3,82 (1 H, m); 4,9—5,3 (2 H, AB-Teile); 5,75—6,05 (1 H, X-Teil); ~ 5,10 (1 H, dxd) δ ppm
MS: wie 2a

IIc IR: 3550, 3460, 1640, 1160, 1110, 1085, 1050, 1030, 1015, 990, 955, 915 $cm^{-1}$
NMR: 1,10 (3 H, s); 1,32 (3 H, s); 1,64 + 1,70 (je 3 H, s); 3,90 (1 H, m); 4,9—5,3 (2 H, AB-Teil); 5,84—6,14 (1 H, X-Teil); ~ 5,10 (1 H, dxd) δ ppm
MS: 238 ($M^+$, 1), 155 (5), 138 (23), 127 (13), 111 (24), 109 (100), 93 (28), 81 (15), 69 (85), 55 (26), 43 (70), 41 (42)

IId IR: 3560, 3460, 1640, 1180, 1110, 1055, 1025, 990, 915 $cm^{-1}$
NMR: 1,24 (3 H, s); 1,32 (3 H, s); 1,62 + 1,68 (je 3 H, s); 3,88 (1 H, m); 4,9—5,3 (2 H, AB-Teil); 5,83—6,12 (1 H, X-Teil); ~ 5,10 (1 H, dxd) δ ppm
MS: wie 2c

2-Methyl-2-vinyl-5-(1′-acetoxy-1′,5′-dimethyl-hex-4′-en-1′-yl)-tetrahydrofurane IIIa—IIId

40,0 g (0,168 Mol) des Alkoholgemisches IIa—IId werden in 70 ml Dimethylanilin gelöst, mit einem Gemisch von 13,2 g (0,168 Mol) Acetylchlorid und 8,6 g (0,084 Mol) Essigsäureanhydrid versetzt und anschließend während 15 Stunden bei 60° gerührt. Das abgekühlte Reaktionsgemisch wird in Diäthyläther aufgenommen, nacheinander mit Wasser, verdünnter Salzsäure, Sodalösung und wieder mit Wasser gewaschen, getrocknet und eingeengt. Es resultieren 46,5 g Acetatgemisch IIIa—IIId, welche anschließend der Pyrolyse unterworfen werden.

Analog werden je 3,0 g des Diastereoisomerengemisches IIa/IIb bzw. IIc/IId in das Acetatgemisch IIIa/IIIb (9 : 1) bzw. IIIc/IIId (9 : 1) übergeführt und die erhaltenen Rohprodukte mit Hilfe der präparativen Gaschromatographie gereinigt.

Spektrale Daten

IIIa/IIIb (9 : 1)
IR: 1738, 1250, 1175, 1130, 1060, 1020, 942, 920, 835 $cm^{-1}$
NMR: 1,30 (3 H, s); 1,40 (3 H, s); 1,60 + 1,69 (je 3 H, s); 2,00 (3 H, s); 4,31 (1 H, m); 4,9—5,3 (2 H, AB-Teil der Vinylgruppe); 5,65—6,12 (1 H, X-Teil der Vinylgruppe); ~ 5,10 (1 H, m) δ ppm
MS: 280 ($M^+$, 1), 151 (67), 138 (100), 111 (98), 109 (90), 96 (30), 93 (51), 81 (30), 69 (50), 55 (21), 43 (85), 41 (37)

IIIc/IIId (9 : 1)
IR: 1738, 1250, 1175, 1062, 1020, 980, 920, 835 $cm^{-1}$
NMR: 1,30 (3 H, s); 1,42 (3 H, s); 1,59 + 1,67 (je 3 H, s); 1,98 (3 H, s); 4,32 (1 H, m); 4,8—5,3 (2 H, AB-Teil); 5,6—6,2 (1 H, X-Teil); ~ 5,10 (1 H, m)
MS: 280 ($M^+$, 1); 151 (62), 138 (96), 111 (100), 109 (95), 96 (32), 93 (54), 81 (32), 69 (53), 43 (95), 41 (42).

2-Methyl-2-vinyl-5-(1′-methylen-5′-methyl-hex-4′-en-1′-yl)-tetrahydrofurane +
2-Methyl-2-vinyl-5-(1′,5′-dimethylhexa-1′,4′-dien-1′-yl)-tetrahydrofurane Ia—Id

Als Pyrolyseapparatur dient ein Quarzrohr von 50 cm Länge und 5 cm Durchmesser, welches mit Glasraschigringen von 2—4 mm Durchmesser gefüllt und mit einem Heizmantel auf eine Temperatur von 400° ±5° konditioniert wird. 46,5 g (0,165 Mol) des Acetatgemisches IIIa—IIId werden in 120 ml Hexan gelöst und im Verlaufe von 2 Stunden unter gleichzeitigem Durchsatz eines Stickstoffstromes von 0,3 l/Min. in das Pyrolyserohr getropft. Das austretende Pyrolysegut wird in einem gekühlten Gefäß kondensiert, mit Natriumbicarbonatlösung gewaschen, getrocknet und eingeengt. Es resultieren 19,0 g Rohprodukt, das Ia, Ib, Ic und Id im Verhältnis von rund 4 : 2 : 2 : 1 enthält

(gaschromatograpischer Nachweis). Die Destillation des Rohproduktes über eine 20-cm-Widmerkolonne liefert 14,5 g olfaktisch gutes Gemisch von Ia—Id vom Sdp. 78—80°/0,05 mm Hg.

Analog werden je 2,5 g des Diastereoisomerengemisches IIIa/IIIb bzw. IIIc/IIId zu Ia + Ic (2 : 1) bzw. Ib + Id (2 : 1) pyrolysiert.

Spektrale Daten

| | | |
|---|---|---|
| Ia | IR: | 3080, 1645, 1235, 1125, 1095, 1050, 1020, 988, 916, 892, 820 $cm^{-1}$ |
| | NMR: | 1,36 (3 H, s), 1,62 + 1,70 (je 3 H, s), 4,40 (1 H, m), 4,82 + 5,10 (je 1 H), 5,1 (1 H, m), 4,9—5,3 (2 H, AB-Teil der Vinylgruppe), 5,75—6,05 (1 H, X-Teil der Vinylgruppe) $\delta$ ppm |
| | MS: | 220 ($M^+$, 6), 152 (37), 137 (39), 119 (32), 109 (94), 93 (52), 81 (46), 69 (100), 67 (62), 55 (37), 41 (87), weitere charakteristische Fragmente bei m/e 135 (25), 107 (47), 95 (50), 68 (37), 43 (35) |
| Ib | IR: | 3080, 1645, 1245, 1122, 1095, 1050, 1020, 988, 915, 894 $cm^{-1}$ |
| | NMR: | 1,34 (3 H, s), 1,64 + 1,70 (je 3 H, s), 4,44 (1 H, m), 4,84 + 5,14 (je 1 H), ~ 5,1 (1 H, m), 4,9—5,3 (2 H, AB-Teil), 5,83—6,12 (1 H, X-Teil), $\delta$ ppm |
| | MS: | 220 ($M^+$, 2), 152 (25), 137 (27), 119 (20), 109 (52), 93 (31), 81 (29), 69 (66), 67 (47), 55 (41), 41 (100), weitere charakteristische Fragmente bei m/e 135 (20), 107 (27), 95 (26), 68 (24), 43 (35) |
| Ic | IR: | 1642, 1123, 1098, 1018, 992, 920, 875 $cm^{-1}$ |
| | NMR: | 1,34 (3 H, s), 1,64 (6 H, 2 s), 1,68 (3 H, s), 1,80 (2 H, m), 1,88 (2 H, m), 2,72 (2 H, dxd, J ~ 6,8 Hz), 4,32 (1 H, m), 4,9—5,3 (2 H, AB-Teil), 5,7—6,0 (1 H, X-Teil), 5,10 (1 H, dxd, 5,43 (1 H, dxd, J ~ 6,8 Hz) $\delta$ ppm |
| | MS: | 205 ($M^+ - CH_3$, 1), 151 (100), 135 (9), 123 (11), 109 (27), 95 (32), 93 (25), 81 (27), 69 (53), 67 (52), 55 (23), 41 (36), weitere charakteristische Fragmente bei m/e 82 (19), 67 (32), 53 (12), 43 (17) |
| Id | IR: | 1642, 1122, 1098, 1058, 1022, 992, 920, 875 $cm^{-1}$ |
| | NMR: | 1,32 (3 H, s), 1,64 (6 H, 2 s), 1,68 (3 H, s), 1,7—2,1 (4 H), 2,72 (2 H, dxd J ~ 7 Hz), 4,36 (1 H, m), 4,9—5,3 (2 H, AB-Teil), 5,8—6,1 (1 H, X-Teil), ~ 5,10 (1 H, dxd), 5,46 (1 H, dxd, J ~ 7 Hz) $\delta$ ppm |
| | MS: | 205 ($M^+ - CH_3$, 1), 151 (100), 135 (10), 123 (12), 109 (36), 95 (46), 93 (36), 81 (48), 69 (96), 67 (86), 55 (54), 41 (78) weitere charakteristische Fragmente bei m/e 82 (29), 67 (65), 53 (24), 43 (40). |

### Beispiel 1a

2-Methyl-2-vinyl-5-(1′-methylen-5′-methyl-hex-4′-en-1′-yl-tetrahydrofurane und
2-Methyl-2-vinyl-5-(1′,5′-dimethyl-hexa-1′,4′-dien-1′-yl)-tetrahydrofurane Ia—If

Zu einer auf —10° gekühlten Lösung von 0,80 g (3,4 mMol) des Diastereoisomerengemisches IIa/IIb in 80 ml Pyridin werden 2,4 ml Phosphoroxychlorid in 8 ml Pyridin so zugetropft, daß eine Reaktionstemperatur von —5°C nicht überschritten wird. Anschließend rührt man während 4 Stunden bei —10°, dann während 20 Stunden bei Raumtemperatur, verdünnt mit 200 ml Hexan, wäscht mit Wasser, verdünnter Salzsäure, Natriumbicarbonatlösung und wieder mit Wasser, trocknet und engt ein. Es verbleiben 0,63 g Rohprodukt, welche Ia und Ic im ungefähren Verhältnis von 3 : 2 enthalten. Bei der säulenchromatographischen Auftrennung an der 40fachen Menge Kieselgel werden mit Hexan/Äther 30 : 1-Fraktionen erhalten, in denen Ia (0,25 g) und Ic (0,14 g) stark angereichert enthalten sind. Zur Charakterisierung dienen zusätzlich mit Hilfe der präparativen Gaschromatographie (Carbowax 20 M) gereinigte Proben. Ia und Ic zeigen die gleichen spektralen Daten wie die gemäß Beispiel 1 erhaltenen Verbindungen. Das NMR-Spektrum von Ia zeigt jedoch in diesem Fall bei 2,74 ppm ein zusätzliches schwaches Signal (≙ ~ 10%) in Form eines dxd (J ~ 7 Hz), obwohl die Probe völlig frei von Ic ist. Ia ist also noch mit rund 10% Ie verunreinigt. Ie läßt sich auf einer Kapillarsäule (50 m UCON) von Ia abtrennen. Das über eine GC-MS-Kopplung aufgenommene Massenspektrum von Ie ist annähernd identisch mit jenem von Ic.

Analog werden 0,80 g des Diastereoisomerenpaares IIc/IId dehydratisiert. Nach Reinigung des Rohproduktes mit Hilfe von Säulenchromatographie und präparativer Gaschromatographie erhält man Ib und Id, welche die gleichen spektralen Daten wie die gemäß Beispiel 1 erhaltenen Verbindungen zeigen. Auch hier zeigt das NMR-Spektrum von Ib eine Verunreinigung (10%) an, bei der es sich dem dxd bei 2,74 ppm (J ~ 7 Hz) zufolge um das Isomere If handelt. Das über eine GC-MS-Kopplung aufgenommene Massenspektrum von If ist annähernd identisch mit jenem von Id.

Auf dieselbe Weise kann das Gemisch IIa—IId (ex 2 : 1-Gemisch von IVa/IVb) bei niedrigen

Temperaturen zum Gemisch Ia—If dehydratisiert werden, also zu einem Gemisch, das auch noch die Isomeren Ie und If (cis-Konfiguration der DB zwischen $C_{1'}$ und $C_{2'}$) enthält.

In den folgenden Beispielen steht »I« vorzugsweise für das Isomerengemisch Ia—Id, wie dies durch Pyrolyse der Acetate IIIa—IIId erhältlich ist.

## Beispiel 2

### a) Fruchtiger Komplex

| | Gewichtsteile |
|---|---|
| Linalylacetat | 500 |
| Dimethylbenzylcarbinylbutyrat | 450 |
| | 950 |

Nach Zugabe von 50 Teilen I wird der Komplex frischer, fruchtiger, natürlicher, es entsteht eine Bergamotteölnote.

### b) Blumig-fruchtiger Komplex

| | Gewichtsteile |
|---|---|
| Linalylacetat | 400 |
| Linalool | 400 |
| | 800 |

Nach Zugabe von 100 Teilen I wird der Komplex süßer, würziger und es entsteht auch hier ein Bergamotteöl-effekt.

### c) Blumiger Komplex

| | Gewichtsteile |
|---|---|
| Phenyläthylalkohol | 400 |
| Benzylacetat | 400 |
| | 800 |

Nach Zugabe von 100 Teilen I wird der Komplex, der vorher etwas spitz war, zu einer blumigen, harmonischen Parfümerie-Base mit leicht rosigem Charakter.

## Beispiel 3

### Parfümerie-Base in Richtung Erdbeere

| | Gewichtsteile |
|---|---|
| Linalylacetat | 300 |
| Benzylacetat | 200 |
| Linalool | 200 |
| Dimethylbenzylcarbinylbutyrat | 200 |
| Bornylacetat | 20 |
| Fructone ® (2-Methyl-1,3-dioxolan-2-äthylacetat) | 10 |
| Maltylisobutyrat | 10 |
| Corps Cassis ® Givaudan (p-Menthan-8-thiol-3-on) | 10 |
| γ-Undecalacton | 10 |
| | 960 |

Gibt man zu dieser Erdbeer-Base 40 Teile I, so wird sie Richtung Walderdbeere modifiziert. Sie wirkt viel aromatischer, frischer und natürlicher.

Beispiel 4

Parfümerie-Base fruchtige Richtung

| | Gewichtsteile |
|---|---|
| Äthyl-3-methyl-3-phenylglycidat | 100 |
| Äthylacetoacetat | 30 |
| Maltylisobutyrat | 30 |
| Dimethylbenzylcarbinylbutyrat | 20 |
| Benzylacetat | 10 |
| Äthylacetat 10% in Propylenglykol | 6 |
| Zitronenöl | 4 |
| | 200 |
| Propylenglykol | 700 |
| Total | 900 |

Der Zusatz von 100 Teilen I zu dieser Base fruchtiger Richtung mit leichtem Erdbeercharakter bewirkt eine völlige Veränderung Richtung Apfel.

Beispiel 5

Parfümerie-Base Richtung Apfel

| | Gewichtsteile |
|---|---|
| Äthyl-3-methyl-3-phenylglycidat | 100 |
| ortho-tert.-butyl-cyclohexylacetat | 40 |
| Äthylacetoacetat | 30 |
| Maltylisobutyrat | 30 |
| Dimethylbenzylcarbinylbutyrat | 20 |
| Benzylacetat | 10 |
| Cyclal ® Givaudan (2,4-Dimethyl-3-cyclo-hexen-1-carboxaldehyd (10% in Propylenglykol) | 10 |
| Äthylacetat (10% in Propylenglykol) | 6 |
| Zitronenöl | 4 |
| | 250 |
| + Propylenglykol | 730 |
| Total | 980 |

Gibt man zur Apfelbase (Richtung gekochte Äpfel) 20 Teile I, so wird diese in Richtung frischem und natürlichem Apfel verändert. Erinnerte die ursprüngliche Base eher an Apfelmus, wiedergibt die neue Base den Geruch der Apfelschale.

Beispiel 6

Parfümerie-Base allgemein blumiger Richtung

| | Gewichtsteile |
|---|---|
| Methyl-dihydro-jasmonat | 200 |
| Linalool | 200 |
| Benzylacetat | 200 |
| Hydroxycitronellal | 100 |
| α-Hexylzimtaldehyd | 100 |
| Citronellol | 60 |
| γ-Undecalacton (10% in Propylenglykol) | 40 |
| Hexenylacetat (10% in Propylenglykol) | 20 |
| Ylang-Öl | 10 |
| Phenyläthylalkohol | 10 |
| Indol (10% in Propylenglykol) | 20 |
| | 960 |

Gibt man zu dieser allgemein blumigen Base 40 Teile I, so wird diese in angenehmer Weise Richtung Ylang und Jasmin verändert. Sie wirkt nun voller und runder.

Beispiel 7

Parfümerie-Base Richtung Melone

| | Gewichtsteile |
|---|---|
| Myraldylacetat | 120 |
| Dimethylbenzylcarbinylbutyrat | 120 |
| Zyklamenaldehyd | 80 |
| Hexenylsalicylat | 100 |
| Fructone ® | 60 |
| Melonal ® (2,6-Dimethyl-5-hepten-1-al) (10% in Propylenglykol) | 60 |
| cis-6-Nonenol (10% in Propylenglykol) | 40 |
| Lilial ® Givaudan (p-tert.-Butyl-$\alpha$-methyl-hydrozimtaldehyd (10% in Propylenglykol) | 20 |
| Zitronenöl | 20 |
| Propylenglykol | 200 |
| | 820 |

Gibt man zu dieser Melonen-Base 180 Teile I, so erhält man eine viel saftiger wirkende Melone. Sie wirkt natürlicher, frischer und kräftiger. Der Eindruck einer Zuckermelone herrscht vor, währenddem die ursprüngliche Base mehr an Wassermelone erinnerte.

Beispiel 8

Apfelaroma

| | Gewichtsteile |
|---|---|
| Maltol | 0,2 |
| Benzaldehyd | 0,3 |
| Citral | 0,3 |
| Vanillin | 0,3 |
| d-Limonen | 0,5 |
| cis-3-Hexenylacetat | 0,5 |
| $\gamma$-Undelacton | 0,5 |
| Capronaldehyd | 1,5 |
| Äthylbutyrat | 4,0 |
| n-Hexanol | 18,0 |
| n-Hexylacetat | 50,0 |
| Äthanol | 901,9 |
| | 980,0 |

Gibt man zu diesem Apfelaroma 20 Teile einer 10%igen Lösung von I in Äthanol, so wird die vorher ausgeprägte estrige Note abgeschwächt. Dafür tritt nun eine angenehme fruchtige Note in Erscheinung. Geschacklich ist bei einer Applikation von 100 g Aroma pro 100 l Zuckersirup, mit Wasser 1 : 5 verdünnt, ebenfalls eine deutliche Verbesserung feststellbar. Ganz im Gegensatz zum ursprünglichen Aroma (konventionelles, fantasieloses Apfelaroma) erinnert nun die angenehme, süße, fruchtige Geschmacksnote an die Apfelsorte »Golden Delicious«.

Beispiel 9

Birnenaroma

| | Gewichtsteile |
|---|---|
| Anethol (10% in Äthanol) | 0,2 |
| Linalylacetat (10% in Äthanol) | 0,2 |
| Vanillin (10% in Äthanol) | 1,0 |
| Hexylacetat | 4,0 |
| Äthyl-2,4-decadienoat | 5,0 |
| Äthylacetat | 5,0 |
| Piperonylacetat | 10,0 |
| Geranylpropionat | 15,0 |
| Isoamylacetat | 50,0 |
| Äthanol | 889,6 |
| | 980,0 |

Gibt man zu diesem Birnenaroma 20 Teile einer 10%igen Lösung von I in Äthanol, so wird die eher aufdringlich wirkende estrige Note zu Gunsten einer angenehm süßen, fruchtigen Note unterdrückt. Geschmacklich ist bei einer Applikation von 100 g Aroma pro 100 l Zuckersirup, mit Wasser 1 : 5 verdünnt, ebenfalls eine deutliche Verbesserung feststellbar. Im Gegensatz zum ursprünglichen konventionellen Aroma kommt nun der Charakter von Tafelbirnen zur Geltung.

**Patentansprüche**

1. Verbindungen der Formel

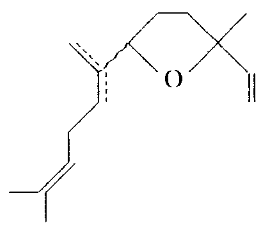

(I)

worin eine der gestrichelt gezeichneten Linien eine zusätzliche Bindung bedeutet.

2. 2-Methyl-2-vinyl-5-(1′-methylen-5′-methyl-hex-4′-en-1′-yl)-tetrahydrofuran.

3. 2-Methyl-2-vinyl-5-(1′,5′-dimethyl-hexa-1′,4′-dien-1′-yl)-tetrahydrofuran.

4. Verbindungen der Formel

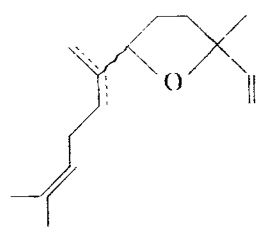

(I)

worin eine der gestrichelt gezeichneten Linien eine zusätzliche Bindung bedeutet, als solche oder in Form von Gemischen, mit der Ausnahme der natürlichen, Verbindungen der Formel I enthaltenden Gemische.

5. Praktisch reine Verbindungen der Formel

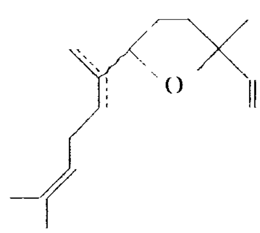

(I)

worin eine der gestrichelt gezeichneten Linien eine zusätzliche Bindung bedeutet.

6. Praktisch reines 2-Methyl-2-vinyl-5-(1′-methylen-5′-methyl-hex-4′-en-1′-yl)-tetrahydrofuran.
7. Praktisch reines 2-Methyl-2-vinyl-5-(1′,5′-dimethyl-hexa-1′,4′-dien-1′-yl)-tetrahydrofuran.
8. Synthetisch hergestellte Verbindungen der Formel

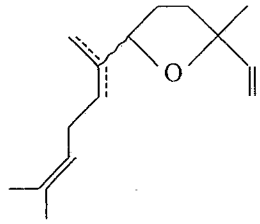

(I)

worin eine der gestrichelt gezeichneten Linien eine zusätzliche Bindung bedeutet.
9. Synthetisch hergestelltes 2-Methyl-2-vinyl-5-(1′-methylen-5′-methyl-hex-4′-en-1′-yl)-tetrahydrofuran.
10. Synthetisch hergestelltes 2-Methyl-2-vinyl-5-(1′,5′-dimethyl-hexa-1′,4′-dien-1′-yl)-tetrahydrofuran.
11. Verbindungen der Formel

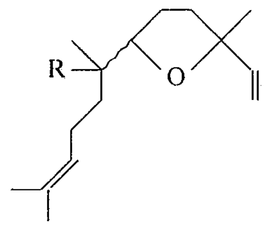

(V)

worin R Hydroxy oder Acyloxy darstellt.
12. 2-Methyl-2-vinyl-5-(1′-hydroxy-1′,5′-dimethyl-hex-4′-en-1′-yl)-tetrahydrofuran.
13. 2-Methyl-2-vinyl-5-(1′-acetoxy-1′,5′-dimethyl-hex-4′-en-1′-yl)-tetrahydrofuran.
14. Verbindungen der Formel

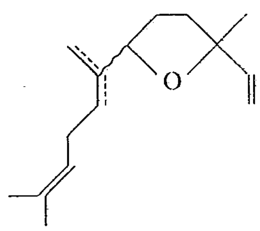

(I)

worin eine der gestrichelt gezeichneten Linien eine zusätzliche Bindung bedeutet, als Riechstoffe.
15. Verfahren zur Herstellung der Verbindungen der Formel

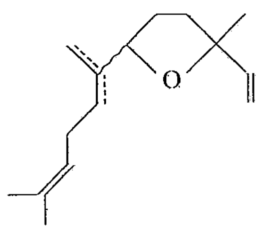

(I)

worin eine der gestrichelt gezeichneten Linien eine zusätzliche Bindung darstellt, dadurch gekennzeichnet, daß man 2-Methyl-2-vinyl-5-(1′-hydroxy (bzw. acyloxy)-1′,5′-dimethyl-hex-4′-en-1′-yl)-tetrahydrofuran dehydratisiert bzw. deacyliert.

16. Riech- und/oder Geschmackstoffkompositionen, gekennzeichnet durch einen Gehalt an einer Verbindung der Formel

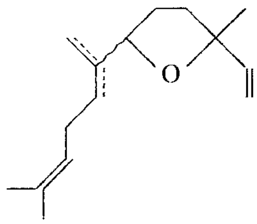

(I)

worin eine der gestrichelt gezeichneten Linien eine zusätzliche Bindung bedeutet, in praktisch reiner Form oder in Form von Gemischen, mit Ausnahme der natürlichen, Verbindungen der Formel I enthaltenden Gemische.

17. Riech- und/oder Geschmackstoffkompositionen nach Anspruch 16, gekennzeichnet durch einen Gehalt an einer synthetisch hergestellten Verbindung der Formel I.

18. Verwendung von Verbindungen der Formel

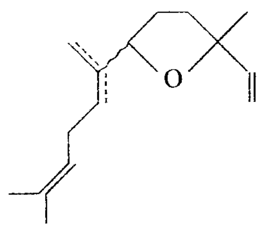

(I)

worin eine der gestrichelt gezeichneten Linien eine zusätzliche Bindung bedeutet, in praktisch reiner Form oder in Form von Gemischen, mit Ausnahme der natürlichen, Verbindungen der Formel I enthaltenden Gemische, als Riech- und/oder Geschmackstoffe.

19. Verwendung von praktisch reinen Verbindungen der Formel

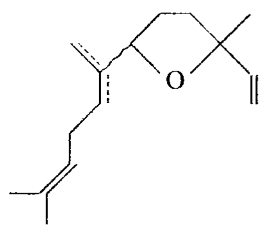

(I)

worin eine der gestrichelt gezeichneten Linien eine zusätzliche Bindung bedeutet, als Riech- und/oder Geschmackstoffe.

20. Verwendung von synthetisch hergestellten Verbindungen der Formel

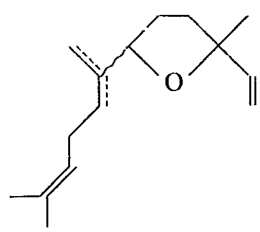

(I)

worin eine der gestrichelt gezeichneten Linien eine zusätzliche Bindung bedeutet, als Riech- und/oder Geschmackstoffe.

**Claims**

1. Compounds of the formula

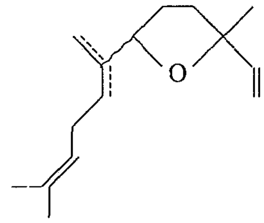

(I)

wherein one of the dotted lines signifies an additional bond.

2. 2-Methyl-2-vinyl-5-(1′-methylene-5′-methyl-hex-4′-en-1′-yl)-tetrahydrofuran.

3. 2-Methyl-2-vinyl-5-(1′,5′-dimethyl-hexa-1′,4′-dien-1′-yl)-tetrahydrofuran.

4. Compounds of the formula

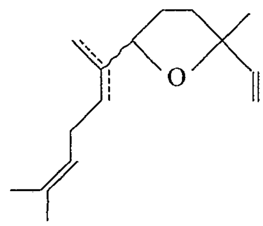

(I)

wherein one of the dotted lines signifies an additional bond, as such or in the form of mixtures, with the exception of natural mixtures containing compounds of formula I.

5. Practically pure compounds of the formula

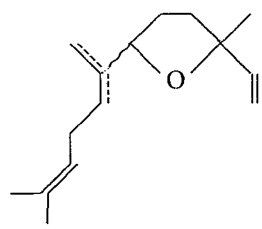

(I)

wherein one of the dotted lines signifies an additional bond.

6. Practically pure 2-methyl-2-vinyl-5-(1′-methylene-5′-methyl-hex-4′-en-1′-yl)-tetrahydrofuran.

7. Practically pure 2-methyl-2-vinyl-5-(1′,5′-dimethyl-hexa-1′,4′-dien-1′-yl)-tetrahydrofuran.

8. Synthetically manufactured compounds of the formula

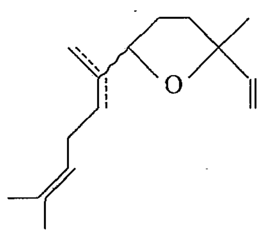

(I)

wherein one of the dotted lines signifies an additional bond.

9. Synthetically manufactured 2-methyl-2-vinyl-5-(1′-methylene-5′-methyl-hex-4′-en-1′-yl)-tetrahydrofuran.

10. Synthetically manufactured 2-methyl-2-vinyl-5-(1′,5′-dimethyl-hexa-1′,4′-dien-1′-yl)-tetrahydrofuran.

11. Compounds of the formula

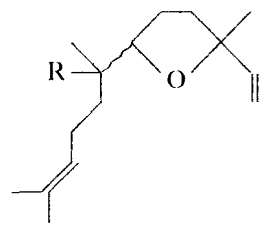

(V)

wherein R represents hydroxy or acyloxy.

12. 2-Methyl-2-vinyl-5-(1′-hydroxy-1′,5′-dimethyl-hex-4′-en-1′-yl)-tetrahydrofuran.

13. 2-Methyl-2-vinyl-5-(1′-acetoxy-1′,5′-dimethyl-hex-4′-en-1′-yl)-tetrahydrofuran.

14. Compounds of the formula

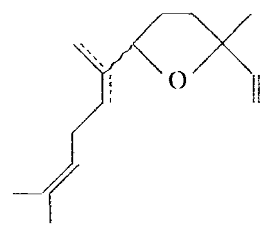

(I)

wherein one of the dotted lines signifies an additional bond, as odorant substances.

15. Process for the manufacture of compounds of the formula

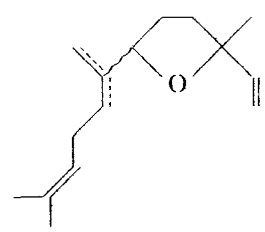

(I)

wherein one of the dotted lines represents an additional bond, characterised in that 2-methyl-2-vinyl-5-(1′-hydroxy(or acyloxy)-1′,5′-dimethyl-hex-4′-en-1′-yl)-tetrahydrofuran is dehydrated or deacylated.

16. Odorant and/or flavouring substance compositions, characterised in that they contain a compound of the formula

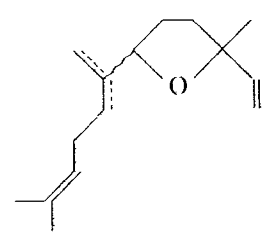

(I)

wherein one of the dotted lines signifies an additional bond, in practically pure form or in the form of mixtures, with the exception of natural mixtures containing compounds of formula I.

17. Odorant and/or flavouring substance compositions according to claim 16, characterised in that they contain a synthetically manufactured compound of formula I.

18. Use of compounds of the formula

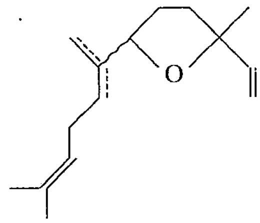

(I)

wherein one of the dotted lines signifies an additional bond, in practically pure form or in the form of mixtures, with the exception of natural mixtures containing compounds of formula I, as odorant and/or flavouring substances.

19. Use of practically pure compounds of the formula

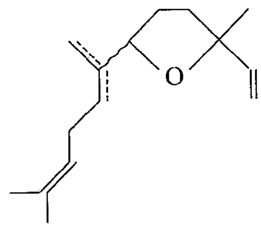

(I)

wherein one of the dotted lines signifies an additional bond, as odorant and/or flavouring substances.

20. Use of synthetically manufactured compounds of the formula

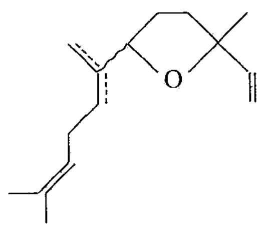

(I)

wherein one of the dotted lines signifies an additional bond, as odorant and/or flavouring substances.

**Revendications**

1. Composés de formule

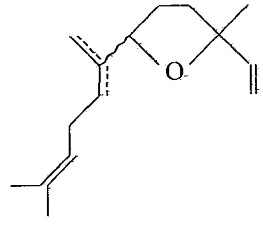

(I)

dans laquelle l'une des lignes en traits interrompus représente une liaison supplémentaire.

2. Le 2-méthyl-2-vinyl-5-(1'-méthylène-5'-méthyl-hexa-4'-ène-1'-yl)-tétrahydrofuranne.

3. Le 2-méthyl-2-vinyl-5-(1',5'-diméthyl-hexa-1',4'-diène-1'-yl)-tétrahydrofuranne.

4. Composés de formule

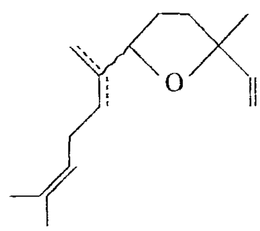

(I)

dans laquelle l'une des lignes en traits interrompus représente une liaison supplémentaire, tels quels ou à l'état de mélanges, à l'exception des mélanges naturels contenant des composés de formule I.

5. Composés pratiquement purs, de formule

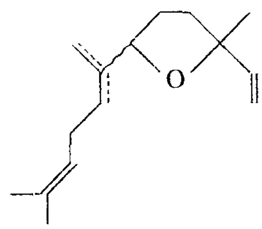

(I)

dans laquelle l'une des lignes en traits interrompus représente une liaison supplémentaire.

6. Le 2-méthyl-2-vinyl-5-(1'-méthylène-5'-méthyl-hexa-4'-ène-1'-yl)-tétrahydrofuranne pratiquement pur.

7. Le 2-méthyl-2-vinyl-5-(1',5'-diméthyl-hexa-1',4'-diène-1'-yl)-tétrahydrofuranne pratiquement pur.

8. Composés préparés par synthèse, de formule

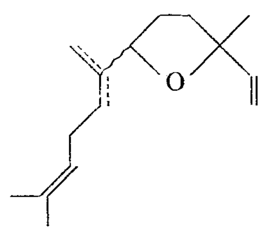

(I)

dans laquelle l'une des lignes en traits interrompus représente une liaison supplémentaire.

9. Le 2-méthyl-2-vinyl-5-(1'-méthylène-5'-méthyl-hexa-4'-ène-1'-yl)-tétrahydrofuranne préparé par synthèse.

10. Le 2-méthyl-2-vinyl-5-(1',5'-diméthyl-hexa-1',4'-diène-1'-yl)-tétrahydrofuranne préparé par synthèse.

11. Composés de formule

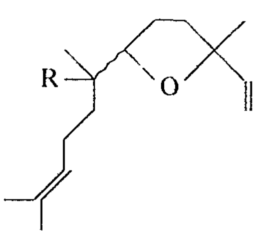

(V)

dans laquelle R représente un groupe hydroxy ou acyloxy.

12. Le 2-méthyl-2-vinyl-5-(1'-hydroxy-1',5'-diméthyl-hexa-4'-ène-1'-yl)-tétrahydrofuranne.

13. Le 2-méthyl-2-vinyl-5-(1'-acétoxy-1',5'-diméthyl-hexa-4'-ène-1'-yl)-tétrahydrofuranne.

14. Composés de formule

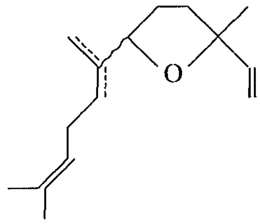

(I)

dans laquelle l'une des lignes en traits interrompus représente une liaison supplémentaire, en tant que parfums.

15. Procédé de préparation des composés de formule

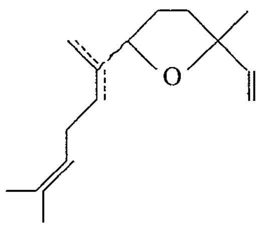

(I)

dans laquelle l'une des lignes en traits interrompus représente une liaison supplémentaire, caractérisé en ce que l'on soumet un 2-méthyl-2-vinyl-5-(1'-hydroxy- ou acyloxy)-1',5'-diméthyl-hexa-4'-ène-1'-yl)-tétrahydrofuranne à déshydratation ou désacylation respectivement.

16. Compositions odoriférantes et/ou aromatisantes caractérisées en ce qu'elles contiennent un composé de formule

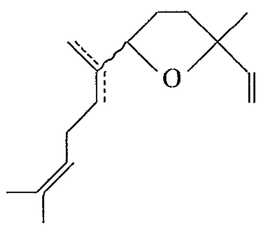

(I)

dans laquelle l'une des lignes en traits interrompus représente une liaison supplémentaire, à l'état pratiquement pur ou à l'état de mélanges, à l'exception des mélanges naturels contenant des composés de formule I.

17. Compositions odoriférantes et/ou aromatisantes selon la revendication 16, caractérisées en ce qu'elles contiennent un composé de formule I préparé par synthèse.

18. Utilisation des composés de formule

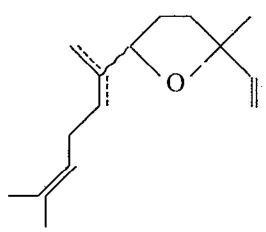

(I)

dans laquelle l'une des lignes en traits interrompus représente une liaison supplémentaire, à l'état pratiquement pur ou à l'état de mélanges, à l'exception des mélanges naturels contenant des composés de formule I, en tant que parfums et/ou arômes.

19. Utilisation des composés de formule

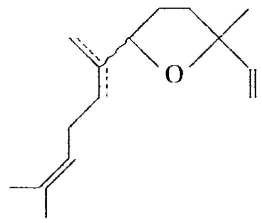

(I)

dans laquelle l'une des lignes en traits interrompus représente une liaison supplémentaire, à l'état pratiquement pur, en tant que parfums et/ou arômes.

20. Utilisation des composés de formule

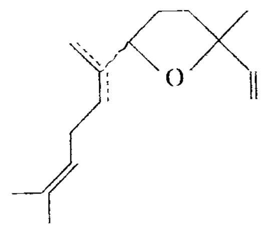

(I)

dans laquelle l'une des lignes en traits interrompus représente une liaison supplémentaire, prépares par synthèse,
en tant que parfums et/ou arômes.